# EUROPEAN PATENT APPLICATION

(11) **EP 2 123 290 A1**
(43) Date of publication of application: **25.11.2009**
(21) Application number: 07738929.4
(22) Date of filing: 19.03.2007
(51) Int. Cl.: A61K 35/74, A23L 1/06, A23L 1/30, A61K 8/99, A61Q 19/02

(54) **MELANOGENESIS INHIBITORY COMPOSITION**

(71) Applicant: Mizkan Group Corporation, Handa-shi, Aichi 475-8585 (JP)
(72) Inventor: TACHIMOTO, Hideki, Handa-shi Aichi 475-0027 (JP)
(74) Representative: Jacobson, Claude
(86) International application number: PCT/JP2007/055484
(87) International publication number: WO 2008/114376

(57) **Abstract**

Provided are a composition for inhibitingmelanogenesis having a high safety and a higher efficacy when the composition is administered internally (oral administration, injection, etc.) or externally (application to skin, patch to skin, etc.), and a skin-lightening method with the use of the composition. Namely, provided are: a composition for inhibiting melanogenesis comprising the intracellular content of an acetic acid bacterium as an active ingredient; and a skin-lightening method comprising internally and/or externally administering the composition.

## Description

### Technical Field

The present invention relates to a composition for inhibiting melanogenesis having an action of preventing skin-darkening caused by sunlight irradiation or the like, and more specifically, to a composition for inhibiting melanogenesis comprising an intracellular content of an acetic acid bacterium as an active ingredient and a skin-lightening method using the composition.

### Background Art

Conventionally, it has been known that the inhibition of melanogenesis in the epidermis of skin is important, to prevent skin-darkening (suntan) caused by ultraviolet irradiation or the like, to prevent skin pigmentation such as blemishes or freckles, and to promote effects of lightening and beautifying skins.
Melanogenesis is caused by significantly promoted ability of melanin production in activated melanocyte, but the mechanism of melanogenesis is not completely clarified. However, it is estimated that melanin is produced as follows: tyrosinase converts tyrosine into dopa and further converts the dopa into dopaquinone; and the dopaquinone is converted to various intermediates, resulting in melanin.

As a result, many cosmetics for inhibitingmelanogenesis have been proposed. For example, cosmetics containing a peroxide such as hydrogen peroxide, sodium peroxide, zinc perborate, magnesium perborate, or sodium perborate have been widely used.
However, those peroxides had problems with storage stability, that is, physical or chemical stability, and the like.

In recent years, cosmetics containing a natural product such as cysteine, hydroxyquinone, kojic acid, or ascorbic acid (vitamin C) have been developed.
However, it cannot be said that those cosmetics had fully satisfactory storage stability and skin-lightening effect.
Note that koj ic acid among the above-mentioned natural products is a substance produced by acetic acid bacteria belonging to *Acetobacter* or *Gluconobacter*, and there have been proposed skin-lightening cosmetics, which contain a cultured broth of an acetic acid bacterium capable of producing kojic acid (cells of the acetic acid bacterium were removed) and have effects of skin-lightening and preventing suntan (refer to Patent Document 1, for example). However, development of a composition for inhibiting melanogenesis having a high safety and a higher efficacy has been further required.

Patent Document 1: JP 53-6432 A

### Disclosure of the Invention

### Problems to be solved by the Invention

An object of the present invention is to provide a composition for inhibiting melanogenesis having a high safety and a higher efficacy when the composition is administered internally (oral administration, injection, etc.) and/or externally (application to skin, patch to skin, etc.), and a method for preventing suntan with the use of the composition for inhibiting melanogenesis.

### Means for solving the Problems

In view of the above problems, the inventor of the present invention focused on acetic acid bacteria which have been used as vinegar-producing bacteria since ancient time and confirmed to have high safety. Note that the acetic acid bacteria have been deeply related to the dietary life of the human since ancient time. For example, in addition to the example of the vinegar production, Caspian yogurt as a traditional food of Europe contains acetic acid bacteria and it is known that the acetic acid bacteria have been eaten historically.
Note that, the acetic acid bacteria are filtered and disposed after the completion of the fermentation in the vinegar manufacturing, and thus is available with inexpensive price.

The inventor of the present invention has confirmed that intracellular contents of such acetic acid bacteria have a strong melanogenesis inhibitory activity, leading to a suntan prevention, thereby completing the present invention.

That is, a first embodiment of the present invention provides a composition for inhibiting melanogenesis, comprising an intracellular content of an acetic acid bacterium as an active ingredient.
In addition, a second embodiment of the present invention provides a composition for inhibiting melanogenesis according to the first embodiment, which is prepared for internal use.
Next, a third embodiment of the present invention provides a composition for inhibiting melanogenesis according to the first embodiment, which is prepared for external use.
Further, a fourth embodiment of the present invention provides a skin-lightening method comprising internally and/or externally administering the composition for inhibiting melanogenesis according to any one of the first to third embodiments.

Note that in the present invention, an intracellular content of an acetic acid bacterium is contained in the composition as an active ingredient, which is different from kojic acid that is produced by acetic acid bacteria in cultured broths and has been conventionally known to have a skin-lightening effect.

### Effect of the Invention

The intracellular content of an acetic acid bacterium of the present invention is administered internally and/or externally, whereby a strong efficacy of inhibiting melanogenesis can be exhibited. Therefore, according to the present invention, a composition for inhibiting melanogenesis having a high safety and a higher efficacy and a skin-lightening method can be provided.

### Brief Description of the Drawing

Fig. 1 is a view illustrating a modified Bronaugh diffusion chamber.

### Best Mode for carrying out the Invention

The acetic acid bacterium used in the present invention is not particularly limited. For example, acetic acid bacteria belonging to *Gluconacetobacter, Gluconobacter, Acetobacter, Asaia,* or *Acidomonas* are exemplified.

More specifically, examples of the acetic acidbacterium belonging to *Gluconacetobacter* include *Gluconacetobacter hansenii, Gluconacetobacter diazotrophicus, Gluconacetobacter intermedius, Gluconacetobacter sacchari, Gluconacetobacter xylinus, Gluconacetobacter europaeus*, and *Gluconacetobacter oboediens.*

Next, examples of the acetic acid bacterium belonging to *Gluconobacter* include *Gluconobacter frateurii* and *Gluconobacter cerinus.*

In addition, examples of the acetic acid bacterium belonging to *Acetobacter* include *Acetobacter tropicalis, Acetobacter indonesiensis, Acetobacter syzygii, Acetobacter cibinongensis, Acetobacter orientalis, Acetobacter pasteurianus, Acetobacter orleanensis, Acetobacter lovaniensis, Acetobacter aceti, Acetobacter pomorum, and Acetobacter malorum*.

Further, examples of the acetic acidbacteriumbelonging to *Asaia* include *Asaia bogorensis* and *Asaia siamensis*.

In addition, examples of the acetic acid bacterium belonging to *Acidomonas* include *Acidomonas methanolica*.

Further, as the acetic acid bacterium, in addition to the above-mentioned bacteria, an acetic acid bacterium used in a vinegar production or a fermented food such as yogurt, an acetic acid bacterium isolated from the nature, and a stock stain which is preserved in microbial culture collections and can be shared are appropriately used.

The acetic acid bacteria are used in the fermentation of acetic acid. In addition, the acetic acid bacteria have been eaten as natadecoco, Caspian yogurt, Kombucha, and the like, and hence are recognized to have high safety. In particular, the acetic acid bacteria are filtered and disposed after the fermentation in the vinegar fermentation, and thus are available in large amount with inexpensive price.

In the first embodiment of the present invention, the composition for inhibiting melanogenesis is comprising the above-mentioned intracellular content of an acetic acid bacterium. The intracellular content may be prepared, for example by using, a suspension which is prepared by subjecting washed cells of an acetic acid bacterium to a disrupting treatment to leak the intracellular content and allowing the cell debris to be contained in a suspended form (hereinafter, may be referred to as a suspension) or by using a supernatant obtained by removing the cell debris through centrifugation of the suspension with a centrifuge (hereinafter, may be referred to as a supernatant).
In addition, a concentrated or dried product of the suspension or supernatant can also be used effectively.

The treatment with a cell disrupter of acetic acid bacteria may be conducted according to a known method, and is conducted, for example, by suspending acetic acid bacteria in distilled water or any of various buffers such as a phosphate buffer and a citrate buffer and disrupting cells using an ultrasonic cell disrupter or a high pressure cell disrupter such as French press.
Note that the above-mentioned suspension or supernatant may be prepared using, in addition to water or various buffers, any of various hydrophilic organic solvents such as acetone, ethanol, methanol, propanol, isopropanol, and butanol in combination.

The melanogenesis inhibitory effect according to the first embodiment of the present invention can be confirmed by the following method.
That is, a test system in which a removed human skin slice is irradiated with UVA to cause melanin pigmentation may be used. In the case of internal use, the test system can examine the effect by applying a substance to be evaluated right beneath the removed human skin slice. Meanwhile, in the case of external use, the test system can examine the effect by applying a substance to be evaluated to the surface of the removed human skin slice.

The production of the composition for inhibiting melanogenesis using an intracellular content of an acetic acid bacterium may be conducted according to a known method. For example, as described above, after cells of the acetic acid bacterium are suspended in distilled water or the like and disrupted using ultrasonic cell disrupter, the suspension is centrifuged to remove cell debris, whereby a supernatant is prepared. Note that, if necessary, the supernatant may be concentrated.

The supernatant or the concentrate can be used directly as a composition for inhibiting melanogenesis. Further, the melanogenesis inhibitory substance is purified by liquid chromatography, operating in a counter current distribution method, or the like, and the purified resultant is collected and can also be used for the composition for inhibiting melanogenesis.

In addition, as the form of the composition for inhibiting melanogenesis according to the first embodiment of the present invention, a solution in which the composition for inhibiting melanogenesis is dissolved in water, an alcohol, a hydrous alcohol, or the like, a powder obtained by drying the solution, or a tablet obtained by molding the powder may be used.

The second embodiment of the present invention is the composition for inhibiting melanogenesis according to the first embodiment, which is prepared for internal use.
In the case of internal use, the composition for inhibiting melanogenesis is used as a supplementary food for cosmetics, food, and drug (oral medicine, injection drug, and the like), and the form thereof is not particularly limited.
As the form of the food in the case of internal use, the composition for inhibiting melanogenesis is mixed in: specifically, a vegetable juice using a tomato, carrot, or the like as a raw material, a fruit juice using an apple, pineapple, grapefruit, orange, peach, or the like as a raw material, a mixture product of a vegetable juice and a fruit juice, an alcoholic beverage, a daily product such as milk and yogurt, a sport beverage, coffee, a tea product such as a black tea, green tea, and Oolong tea, a vinegar such as a black vinegar, a grain vinegar, a rice vinegar, an unpolished-rice vinegar, a black vinegar,aspirit vinegar, a fruit vinegar containing a fruit as a raw material such as an apple cider vinegar and a wine vinegar, and a vegetable vinegar containing a vegetable as a raw material, a confectionery such as a candy, gum, jelly, ice cream, and cookie, a staple food such as bread, rice, and noodles, or the like.

The third embodiment of the present invention is a composition for inhibiting melanogenesis according to the first embodiment, which is prepared for external use.
In the case of external use, the composition may be used as a skin patch or application agent, and the form thereof is not particularly limited.
For example, as the form of the application agent for external use, the composition is mixed in, specifically, a basic cosmetics such as a lotion, cosmetic cream, milky lotion, or pack; a makeup cosmetic such as a foundation; a toiletry product such as a shampoo, rinse, or body shampoo; or a bath agent, all of which is blended respectively with a vehicle such as glycerol or cellulose; a thickener such as carboxymethylcellulose or gum arabic; and a surfactant such as polyoxyethylene or sorbitan fatty acid ester.

When the composition for inhibiting melanogenesis of the present invention is prepared in the above-mentioned form, another functional component derived from a natural product is contained, whereby an additive effect or a synergistic effect may also be expected.

The dosage of the intracellular content of an acetic acid bacterium as the composition for inhibiting melanogenesis of the present invention depends on the concentration of an active component in the intracellular content. In general, in the case of the composition for internal use, the dosage is 0.001 mg to 100 g and preferably 0.1 mg to 10 g per adult per day.
In the case of the composition for external use, the dosage is 0.001 mg to 100 g and preferably 0.01 mg to 10 g each 100 cm² of skin.
In the case of simultaneous administration of the composition for internal use and the composition for external use, the dose of the composition for internal use is 0.001 mg to 100 mg per adult per day, and the dose of the composition for external use is 0.001 mg to 100 g each 100 cm² of skin. Preferably, the dose of the composition for internal use is 0.01 mg to 10 g per adult per day, and the dose of the composition for external use is 0.01 mg to 10 g each 100 cm² of skin.

The fourth embodiment of the present invention is a skin-lightening method comprising internally and/or externally administering the composition for inhibiting melanogenesis according to any one of the first to third embodiments.
To prevent suntan and lighten the skin, the composition for inhibiting melanogenesis according to any one of the first to third embodiments may be used internally or externally, or the composition for internal use and the composition for external use may be used in combination.
In the case where the composition is used internally and/or externally, the dosage of the intracellular content of an acetic acid bacterium or the like is as described above.
In the fourth embodiment of the present invention, it is possible to prevent suntan by using the composition for inhibiting melanogenesis according to any one of the first to third embodiments internally and/or externally.

### Examples

Hereinafter, the present invention is described in detail by way of examples, but the present invention is not limited thereby.

### Example 1 (Preparation of suspension and supernatant in large amount)

As an acetic acid bacterial strain, *Gluconacetobacter xylinus* NBRC15237 strain was used. The acetic acid bacterial strain is preserved in National Institute of Technology and Evaluation, Department of Biotechnology, Biological Resource Center (2-5-8, Kazusakamatari, Kisarazu-shi, Chiba-ken) and the acetic acid bacterium can be shared.
Cells in 10 kL of acetic acid-fermented broth, which was obtained by acetic acid fermentation using the acetic acid bacterium, were collected with a high speed centrifuge (8,000 rpm, 20 minutes), whereby 10 kg of wet cells were obtained. 10 kg of the obtained wet cells were suspended in 35 L of water, and disrupted using a French press cell disrupter at 10,000 psi, to thereby obtain a suspension.
Thereafter, the cell debris in 40 kg of the obtained suspension were removed with a high speed centrifuge (8, 000 rpm, 20 minutes), to thereby obtain 30 kg of a supernatant.

### Example 2 (Test on melanogenesis inhibitory effect assuming internal use)

To evaluate the efficacy of the suspension or supernatant prepared in Example 1 as an internal agent, the melanogenesis inhibitory effect of the suspension or supernatant was estimated according to the following procedure (1) to (4).

(1) A removed human skin slice (informed consent was obtained) was placed in a modified Bronaugh diffusion chamber (outlined in FIG. 1). The suspension or supernatant prepared in Example 1 was added at a predetermined concentration to a culture medium under the skin slice.
(2) Three hours after the addition of the suspension or supernatant, the skin slice was irradiated with 20 J/cm² of ultraviolet A (UV-A) for 40 seconds. Note that a silicate glass was placed between the lamp and the sample.
(3) Organ culture of the skin slice was conducted. Note that DMEMab medium (+) 10% FBS (fetal bovine serum) was used as a culture medium.
(4) After 24 hours, the degree of melanogenesis was determined by DOPA staining based on a histogram analysis (n=2 to 3). Note that the concentration of melanin is represented as DOPA staining degree (pixel)/length (mm) of basal cell layer in epidermis.

The results are shown in Tables 1 and 2.
As a result, in the test system assuming internal use, it has been revealed that the suspension and supernatant have the melanogenesis inhibitory action.

**[Table 1]**

| Test on melanogenesis inhibitory effect of suspension assuming internal use | | | |
|---|---|---|---|
| Test group | UV | Suspension | Concentration of melanin |
| 1 | Not irradiated | Not administered | 545±34 |
| 2 | Irradiated | Not administered | 8,267±523 |
| 3 | Irradiated | 0.05% (w/v) | 5,828±514 |
| 4 | Irradiated | 0.1% (w/v) | 4,244±301 |
| 5 | Irradiated | 0.5% (w/v) | 3,698±485 |

**[Table 2]**

| Test on melanogenesis inhibitory effect of supernatant assuming internal use | | | |
|---|---|---|---|
| Test group | UV | Supernatant | Concentration of melanin |
| 6 | Not irradiated | Not administered | 367±42 |
| 7 | Irradiated | Not administered | 5,011±679 |
| 8 | Irradiated | 0.05% (w/v) | 3,621±133 |
| 9 | Irradiated | 0.1% (w/v) | 1,560±358 |
| 10 | Irradiated | 0.5% (w/v) | 2,677±409 |

### Example 3 (Test on melanogenesis inhibitory effect assuming external use)

To evaluate the efficacy of the suspension or supernatant prepared in Example 1 as an external agent, the melanogenesis inhibitory effect of the suspension or supernatant was estimated according to the following procedure (1) to (4).

(1) A removed human skin slice (informed consent was obtained) was placed in a modified Bronaugh diffusion chamber (outlined in FIG. 1). The suspension or supernatant prepared in Example 1 was dissolved in water at a predetermined concentration and applied onto the surface of the skin slice.
(2) Three hours after the addition of the suspension or supernatant, the skin slice was irradiated with 20 J/cm² of ultraviolet A (UV-A) for 40 seconds. Note that a silicate glass was placed between the lamp and the sample.
(3) Organ culture of the skin slice was conducted. Note that DMEMab medium (+) 10% FBS (fetal bovine serum) was used as a culture medium.
(4) After 24 hours, the degree of melanogenesis was determined by DOPA staining based on a histogram analysis (n=2 to 3). Note that the concentration of melanin is represented as DOPA staining degree (pixel)/length (mm) of basal cell layer in epidermis.

The results are shown in Tables 3 and 4.
As a result, it has been revealed that in not only internal administration but also external administration, the suspension and supernatant have the melanogenesis inhibitory action.

**[Table 3]**

| Test on melanogenesis inhibitory effect of suspension assuming external use | | | |
|---|---|---|---|
| Test group | UV | Suspension | Concentration of melanin |
| 11 | Not irradiated | Not administered | 662±171 |
| 12 | Irradiated | Not administered | 5,422±728 |
| 13 | Irradiated | 0.5% (w/v) | 3,965±405 |
| 14 | Irradiated | 1% (w/v) | 3,601±241 |
| 15 | Irradiated | 10% (w/v) | 3,646±694 |

**[Table 4]**

| Test on melanogenesis inhibitory effect of supernatant assuming external use | | | |
|---|---|---|---|
| Test group | UV | Supernatant | Concentration of melanin |
| 16 | Not irradiated | Not administered | 910±76 |
| 17 | Irradiated | Not administered | 6,405±839 |
| 18 | Irradiated | 0.5% (w/v) | 4,781±730 |
| 19 | Irradiated | 1% (w/v) | 3,716±706 |
| 20 | Irradiated | 10% (w/v) | 4,317±534 |

### Example 4 (Test on melanogenesis inhibitory effect assuming internal and external combination administration)

To evaluate the efficacy of the suspension or supernatant prepared in Example 1 as combined use of an internal agent and an external agent, the melanogenesis inhibitory effect of the suspension or supernatant was estimated according to the following procedure (1) to (4).

(1) A removed human skin slice (informed consent was obtained) was placed in a modified Bronaugh diffusion chamber (outlined in FIG. 1). The supernatant prepared in Example 1 was added at a predetermined concentration to a culture medium under the skin slice (for internal use). Meanwhile, the suspension prepared in Example 1 was dissolved in water at a predetermined concentration and applied onto the surface of a skin slice (for external use). In addition, as a combination administration test, the supernatant prepared in Example 1 was added at a predetermined concentration to a culture medium under a skin slice (for internal use), while the suspension prepared in Example 1 was dissolved in water at a predetermined concentration and applied onto the surface of the skin slice (for external use).
(2) Three hours after the addition of the suspension and supernatant, the skin slice was irradiated with 20 J/cm² of ultraviolet A (UV-A) for 40 seconds. Note that a silicate glass was placed between the lamp and the sample.
(3) Organ culture of the skin slice was conducted. Note that DMEMab medium (+) 10% FBS (fetal bovine serum) was used as a culture medium.
(4) After 24 hours, the degree of melanogenesis was determined by DOPA staining based on a histogram analysis (n=2 to 3). Note that the concentration of melanin is represented as DOPA staining degree (pixel)/length (mm) of basal cell layer in epidermis.

The results are shown in Table 5.
The results shown in Table 5 have revealed that single administration of the suspension alone or supernatant alone provides the melanogenesis inhibitory effect and that combination administration provides a more remarkable melanogenesis inhibitory effect.

**[Table 5]**

| Test on melanogenesis inhibitory effect assuming internal and external combination administration | | | | |
|---|---|---|---|---|
| Test group | UV | Suspension | Supernatant | Concentration of melanin |
| 21 | Not irradiated | Not administered | Not administered | 801±130 |
| 22 | Irradiated | Not administered | Not administered | 8,657±512 |
| 23 | Irradiated | Not administered | 0.1% (w/v) (Internal use) | 4,610±814 |
| 24 | Irradiated | 1% (w/v) (External use) | Not administered | 5,721±478 |
| 25 | Irradiated | 1% (w/v) (External use) | 0.1% (w/v) (Internal use) | 2,396±403 |

### Example 5 (Production of tablet)

Cells in 10 kL of acetic acid-fermented broth were collected with a high speed centrifuge (8,000 rpm, 20 minutes), whereby 10 kg of wet cells were obtained. 10 kg of the wet cells were suspended in 35 L of water, and disrupted using a French press cell disrupter at 10,000 psi, to thereby obtain an acetic acid bacterium suspension. Thereafter, the cell debris in 40 kg of the obtained acetic acid bacterium suspension was removed with a high speed centrifuge (8, 000 rpm, 20 minutes), to thereby obtain 30 kg of a supernatant. 30 kg of the obtained supernatant was freeze-dried to solidness under reduced pressure with a large freeze-drying apparatus, whereby about 50 g of a dry solid were obtained. 1 g (0.7 wt%) of the obtained dry solid was added with 35 g (26.9 wt%) of crystalline cellulose, 67 g (51.5 wt%) of dry corn starch, 22 g (16.9 wt%) of lactose, 2 g (1.5 wt%) of calcium stearate, and 3 g (2.3 wt%) of polyvinyl pyrrolidone as a binder agent to produce a mixed powder. The obtained powder was filled in a gelatin hard capsule.
It is expected that the tablet prepared above can be used effectively for oral ingestion as a composition for inhibiting melanogenesis.

### Example 6 (Production of jelly)

Cells in 10 kL of acetic acid-fermented broth were collected with a high speed centrifuge (8,000 rpm, 20 minutes), whereby 10 kg of wet cells were obtained. 10 kg of the obtained wet cells were suspended in an equivalent amount of distilled water. 20 kg of the suspension of the acetic acid bacterium was subjected to cell disrupting treatment by being passed through a high pressure homogenizer (20,000 psi) three times to obtain a suspension. After that, the suspension was freeze-dried to solidness under reduced pressure with a large freeze-drying apparatus. Thus, 1.5 kg of a dry solid was obtained.
8 g (0.8 wt%) of the obtained dry solid, 250 g (25 wt%) of sugar, 1.6 g (0.16 wt%) of carageenan, 0.8 g (0.08 wt%) of locust bean gum, and 0.8 g (0.08 wt%) of xanthane gum were uniformly mixed, whereby a powder mixture was obtained. 300 g of water were measured in a pot, 30 g (3.0 wt%) of brown sugar were dissolved therein, and 150 g (15 wt%) of a hydrogenated syrup were mixed therein, and the powder mixture previously obtained was mixed and stirred until no lumps remained, followed by further stirring. Those compounds were stirred uniformly and 258.8 g of the additional water were added thereto, followed by heating. Those compounds were dissolved by heating at the temperature of 85°C for 10 minutes. After that, the resultant was cooled with running water, whereby jelly food was obtained.
It is expected that the jelly prepared above can be used effectively for oral ingestion as a composition for inhibiting melanogenesis.

### Example 7 (Production of beverage)

Cells in 10 kL of acetic acid-fermented broth were collected with a high speed centrifuge (8,000 rpm, 20 minutes), whereby 10 kg of wet cells were obtained. 10 kg of the obtained wet cells were suspended in 100 L of vinegar. The suspension of the acetic acid bacterium was subjected to cell disrupting treatment by being passed through a high-pressure homogenizer (20,000 psi) three times. The obtained suspension was dispensed in 500-ml volume bottles, and the bottle was sterilized by heating to 75°C. Thus, a vinegar beverage was obtained.
It is expected that the beverage prepared above can be used effectively for oral ingestion as a composition for inhibiting melanogenesis.

### Example 8 (Production of lotion)

Cells in 10 kL of acetic acid-fermented broth were collected with a high speed centrifuge (8,000 rpm, 20 minutes), whereby 10 kg of wet cells were obtained. 10 kg of the wet cells were suspended in 35 L of water, and disrupted using a French press cell disrupter at 10,000 psi, to thereby obtain a suspension. Thereafter, the cell debris in 40 kg of the obtained suspension was removed with a high speed centrifuge (8,000 rpm, 20 minutes), to thereby obtain 30 kg of a supernatant. 1 g (2.0 wt%) of the obtained supernatant, 1.5 g (3.0 wt%) of glycerin, 2.5 g (5.0 wt%) of 1,3-butylglycol, 4.0 g (8.0 wt%) of quince seed extract, and 37.7 g (75.5 wt%) of purified water were mixed, and a mixture solution of 0.6 g (1.2 wt%) of polyoxyethylene sorbitan laurate, 2.5 g (5.0 wt%) of ethanol, 0.1 g (0.2 wt%) of methyl p-oxybenzoate, and 0.05 g (0.1 wt%) of a flavor was further added, whereby a lotion was prepared.
It is expected that the lotion prepared above can be applied effectively as a composition for inhibiting melanogenesis.

### Example 9 (Production of cosmetic cream)

Cells in 10 kL of acetic acid-fermented broth were collected with a high speed centrifuge (8,000 rpm, 20 minutes), whereby 10 kg of wet cells were obtained. 10 kg of the obtained wet cells were suspended in an equivalent amount of distilled water. 20 kg of the suspension of the acetic acid bacterium was subjected to cell disrupting treatment by being passed through a high pressure homogenizer (20,000 psi) three times. After that, the resultant was freeze-dried to solidness under reduced pressure with a large freeze-drying apparatus. Thus, 1.5 kg of a dry solid was obtained.
10 g (10.0 wt%) of the obtained dry solid, 0.1 g (0.1 wt%) of xanthan gum, 1.0 g (1.0 wt%) of glycerin, 5.0 g (5.0 wt%) of 1,3-butylene glycol, 0.2 g (0.2 wt%) of methyl p-oxybenzoate, and 55.0 g (55.0 wt%) of purified water were mixed with heating at 75°C, and a solution obtained by dissolving 3.0 g (3.0 wt%) of jojoba oil, 2.0 g (2.0 wt%) of squalene, 0.5 g (0.5 wt%) of methylpolysiloxane, 0.5 g (0.5 wt%) of stearyl alcohol, 12.5 g (12.5 wt%) of glycerol tricaproate, 0.5 g (0.5 wt%) of glycerol monostearate, 1.5 g (1.5 wt%) of diglycerol monostearate, 3.0 g (3.0 wt%) of decaglycerol monostearate, and 0.1 g (0.1 wt%) of propyl p-oxybenzoate with heating at 75°C was mixed therein to emulsify the solution. Then, the emulsion was cooled with stirring to 50°C, and 0.1 g (0.1 wt%) of any of various flavors was added, whereby a cosmetic cream was prepared. It is expected that the cosmetic cream prepared above can be applied effectively as a composition for inhibiting melanogenesis.

### Industrial Applicability

The intracellular content of an acetic acid bacterium of the present invention is administered internally and/or externally, whereby a strong melanogenesis inhibitory effect can be exhibited. Therefore, according to the present invention, a composition for inhibiting melanogenesis having a high safety and a higher efficacy and a skin-lightening method can be provided.
Accordingly, the present invention can be used widely in the fields of foods, beauty products and cosmetics.

## Claims

1. A composition for inhibiting melanogenesis, comprising an intracellular content of an acetic acid bacterium as an active ingredient.

2. A composition for inhibiting melanogenesis according to Claim 1, which is prepared for internal use.

3. A composition for inhibiting melanogenesis according to Claim 1, which is prepared for external use.

4. A skin-lightening method comprising internally and/or externally administering the composition for inhibiting melanogenesis according to any one of Claims 1 to 3.
